# EUROPEAN PATENT SPECIFICATION

(11) **EP 2 433 595 B1**
(45) Date of publication and mention of the grant of the patent: **12.09.2018**
(21) Application number: 10785772.4
(22) Date of filing: 20.05.2010
(51) Int. Cl.: A61F 13/02, A61M 27/00, A61L 15/16

(54) **IMPROVED VACUUM DRESSING FOR USE AS POST-OPERATIVE COMPRESSION TREATMENT**
VERBESSERTE VAKUUMSAUFLAGE ZUR VERWENDUNG BEI DER POSTOPERATIVEN DRUCKBEHANDLUNG
PANSEMENT SOUS VIDE AMÉLIORÉ UTILISABLE COMME TRAITEMENT DE COMPRESSION POSTOPÉRATOIRE

(30) Priority: 20.05.2009 ES 200900931 U
(43) Date of publication of application: 28.03.2012
(73) Proprietor: Marquez Cañada, Juan, 46701 Gandia (Valencia) (ES)
(72) Inventor: Marquez Cañada, Juan, 46701 Gandia (Valencia) (ES)
(74) Representative: Escamilla Condés, Mónica
(86) International application number: PCT/ES2010/000221
(87) International publication number: WO 2010/142819

(56) References cited:
- WO-A1-2009/002260
- ES-T3- 2 151 925
- ES-T3- 2 206 979
- ES-T3- 2 223 977
- ES-T3- 2 261 397
- ES-T3- 2 304 730
- US-A1- 2004 030 304
- US-A1- 2005 090 787
- US-A1- 2006 041 247
- US-A1- 2008 132 819
- US-A1- 2008 215 020

## Description

### OBJECT OF THE INVENTION

The invention, as stated in the present specification, relates to an improved vacuum dressing applicable as post-operative compression treatment, which provides the function it is intended for with several advantages and innovative characteristics, which will be recorded in detail further below, thanks to which it is configured as an improved alternative to the systems currently known in the market for said purpose.

More in particular, the object of the invention centres on a vacuum dressing, of the type which incorporates under its sealing film an external layer of foam with macro pores and a semi-permeable internal layer, whose structural design makes it suitable to be applied directly on the skin surface as post-operative compression treatment, unlike the vacuum dressings traditionally used. With the possibility furthermore, of incorporating a drainage device with modified redon catheters to directly transmit the negative pressures from the surgical plane to the foam layer with open macro pores of the dressing.

### FIELD OF APPLICATION OF THE INVENTION

The field of application of the present invention lies within the technical sector of the industry dedicated to the manufacturing of medical healthcare material, particularly dressings, bandages and similar devices for wounds and/or surgical planes.

### BACKGROUND OF THE INVENTION

The application of vacuum pressure on the bed of a wound has demonstrated, both experimentally and clinically, to accelerate the tissue repair processes. One example of such an application is disclosed in document WO 2009/002260. At present, one of the most used devices for the vacuum-assisted closure of wounds is the "VAC®" system (vacuum-assisted closure, manufactured by KCI, San Antonio, Tex.).

This system consists of placing a sterile reticulated polyurethane foam dressing, with open pores of a big size (400-600 µm) covering the wound throughout its surface. On this sponge is applied a suction tube that is connected to a programmable vacuum pump. The vacuum pump is equipped with a tank for storing the fluid extracted from the wound. The sponge and the outlet of the tube are sealed and isolated from the outside by an adhesive film that is adhered to the surrounding skin, creating a closed system. The high porosity of the polyurethane foam dressing used for this system, due to its regular structure of open macropores, means that the pressure distributed throughout the surface of the wound is uniform; for this, it is essential to guarantee that the system is hermetic and that there are no leaks through the isolating plastic dressing.

According to the manufacturer (KCI), there will be two mechanisms, the macrotension (at tissue level) and the microtension (at cellular level) which will act together to promote healing of the wounds. These mechanisms would be caused by the application of negative pressure and the direct contact between the foam with open pores and the bed of the wound.

However, although it is true that the application of the vacuum therapy on the body surface generates negative pressure in the dressing pores, it is no less important that in the points of contact of the solid components of the foam (pore walls) with the bed of the wound, the system generates positive pressures. This would allow us to expand the indications of this vacuum therapy, not only to promote healing in substance losses, but also as a tool in those cases where post-operative compression is required, such as:
- Liposuction in the areas of difficult compression with the garments of pressure therapy/ conventional supports (e.g. cervicodorsal lipomatosis, localized lipomatosis in morbid obesities, etc.).
- Surgical interventions in general (e.g. abdominoplasties) to minimize the risk of post-operative morbidity, since this device, compared with the conventional support/bandages, constitutes a selective compression which, in addition to increasing the patient's comfort, makes it possible to decrease the stress in the suture lines; which will facilitate the immediate post-operative mobilization of the patient.

However, this macropore dressing has been designed to be applied only on losses of substance/ulcers and should not come into contact with healthy skin. Indeed, the most frequent complication of the system is the irritation and maceration of the skin which, accidentally, may remain in contact with this foam. Therefore, it is necessary to interpose between this macropore polyurethane sponge and the skin surface a type of atraumatic dressing, semi-permeable which protects the healthy skin. There are multiple dressings in the market which satisfy these conditions (Tielle®, Mepilex®, Biatain®, Epifoam®, skinfoam®, allevyn®, therafoam®).

These dressings, generally, are composed of three layers:
- An external layer of polyurethane permeable to water vapour and impermeable to fluids.
- Hydropolymeric central middle layer with a great absorption capacity (generally of polyurethane foam)
- And a microperforated internal layer (adherent or not) of polyurethane or silicon.

However, said dressings, as with the VAC® foam, as they have not been conceived as post-operative compression treatment, have the drawback that their available dimensions, especially in the case of large operating areas, do not make them suitable for this purpose, and although for their use in this indication, as part of a selective vacuum compression system, there is the possibility of coupling them together, the procedure is not very effective, it is tedious and supposes lengthening the surgical procedure. Furthermore, the risks of overlapping or shearing between them and/or with the VAC® foam, prevent maintaining them in situ more than 48 hours to avoid possible adverse effects.

It would, therefore, be desirable, and this is the essential objective of the present invention, to have the creation of a new integrated foam dressing, which avoids the aforementioned drawbacks, i.e. which, being suitable for surgical wounds that require post-operative compression and suitable for being in contact with healthy skin, have dimensions suitable for greater surgical planes and with sufficient thickness to optimize the operative compression and which makes it possible to incorporate a drainage device with redon catheters modified to directly transmit negative pressures from the bloody area underlying the wound until the foam component with macro pores of the dressing. Furthermore, the fact that it is an integrated dressing and that its innermost layer can be adherent, would increase the effectiveness thereof, since it would permit developing negative tangential pressures not only in the periphery of the dressing (due to the external adhesive film which adheres it to the surrounding skin), but also central negative tangential pressures under the adherent surface of the dressing, providing a greater effect of skin retraction and a decrease of tension in the suture lines.

However, the applicant does not know of the existence of any which have said characteristics.

### EXPLANATION OF THE INVENTION

Thus, the improved vacuum dressing applicable as post-operative compression treatment proposed by the present invention, is configured as a novelty within its field of application since, in accordance with its implementation, the objectives previously indicated as suitable are satisfactorily reached, the characterizing details being those that distinguish them, suitably set down in the end claims accompanying the present specification.

In particular, the invention centres on an improved vacuum dressing applicable for surgical planes that require post-operative compression, which comprises an external foam layer with open pores of a big size or macro pores, and a semi-permeable internal layer, said internal layer being adherent or not, and whereon, in conventional manner, a suction tube will be applied connected to a vacuum pump, the outlet of said tube and the dressing being sealed by an adhesive film that adheres to the surrounding skin.

Furthermore, the greater thickness of the foam layer with macro pores enhances the positive sagittal pressures, providing a greater compression effect and also allowing it to additionally incorporate a drainage device consisting of modified redon catheters whereto multiple holes have been made at its two ends, and which directly transmit the negative pressures from the surgical plane to the layer of macropore foam, promoting tissue adhesion and avoiding the placement of external redon catheters.

Finally, it should be highlighted that the fact that it is an integrated dressing and that its internal layer is adherent, makes it possible to develop negative tangential pressures not only in the periphery of the dressing (by the adhesive film which adheres it to the surrounding skin), but also negative tangential pressures (transferred from the foam component with macro pores to the skin surface, through the internal adherent layer of hidropolimeric foam, something which is not possible with the conventional systems).

The advantages provided by the recommended dressing are therefore verified, since, with it, it combines a greater speed of placement and a greater safety and comfort for the patient, improving the efficacy and effectiveness, since, in addition to being able to be directly applied on the skin surface, it can be maintained during a greater period of days than with the current dressing coupling system, as it allows the distribution of uniform pressure in the application area (without risks of hyper or hypopressure derived from the overlapping or shearing between the juxta/superimposed dressings providing a greater effect of skin retraction and a decrease of stress in the suture lines.

The described improved vacuum dressing applicable as post-operative compression treatment therefore represents an innovative structure of structural and constitutive characteristics unknown to now for said purpose, reasons that, together with its practical use, give it sufficient grounds to obtain the privilege of exclusiveness requested.

### DESCRIPTION OF THE DRAWINGS

To complement the description being made and in order to aid towards a better understanding of the characteristics of the invention, a set of plans is attached to the present specification as an integral part thereof, wherein, with illustrative and non-limiting character, the following has been represented:
Figure number 1.- Shows a schematized and disproportionately enlarged representation to facilitate the understanding, of a view of the improved vacuum dressing applicable for surgical planes that require post-operative compression, object of the invention, wherein we can observe the main parts and elements it comprises, as well as the configuration and disposal thereof.
Figure number 2. - Shows a view similar to the above, wherein the drainage device has been incorporated.

### PREFERRED EMBODIMENT OF THE INVENTION

In light of said figures, and in accordance with the numbering adopted, it is possible to observe in them an example of preferred embodiment of the invention, which comprises the parts and elements indicated and are described in detail below

Thus, as can be observed in said figures, the dressing (1) in question is sealed by an adhesive film (5) which adheres to the skin (6) surrounding the surgical wound (7) and the area of surgical detachment (8), placing on this adhesive film a suction tube (4) connected to a vacuum pump (not represented).

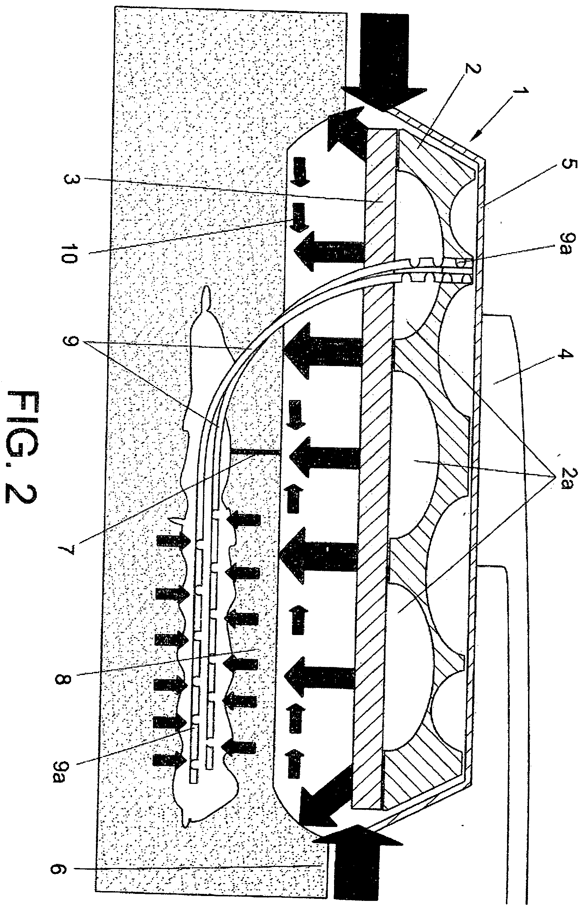

This improved vacuum dressing, under its sealing film, is essentially configured from two layers (2 and 3):
- an external layer (2) of foam with open pores (2a) of a big size or macro pores.
- and an internal layer (3) semi permeable, which is adherent.
Wherein the external layer (2) has the special characteristic of having a certain thickness that favours more effective compression and the possibility of incorporation of a drainage device which, as is observed in figure 2, consists of redon catheters or tubes (9) whereto a plurality of holes (9a) has been made at its two ends, of which ends, one is inserted in the external layer (2) of foam with macro pores, and the opposite is designed to be inserted in the surgical plane (8), so that said tubes (9) directly transmit the negative pressures from said plane to the external layer (2) of foam with macro pores, promoting tissue adhesion.

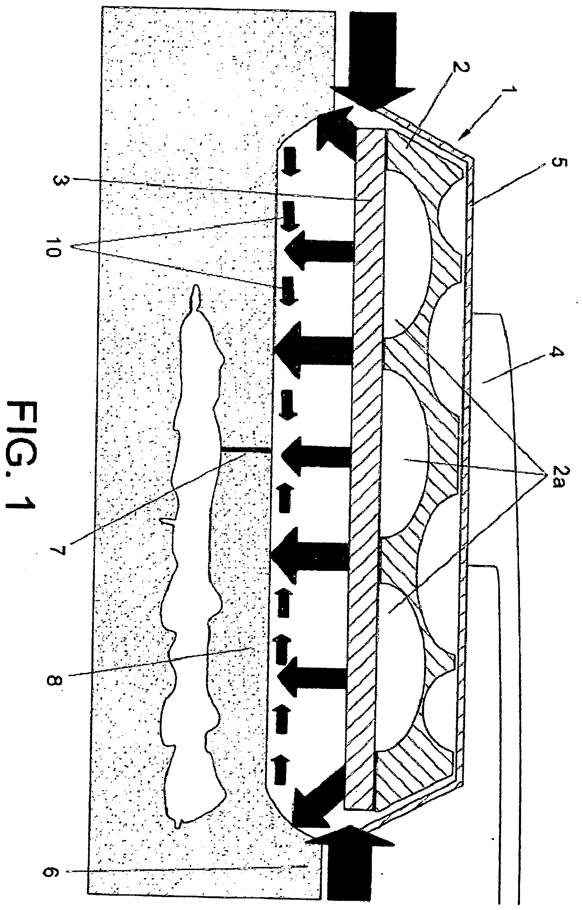

Both figure 1 and figure 2 show with arrows the positive pressures or sagittal compression exerted by the external (2) and internal (3) layers of the dressing (1), the negative tangential pressures exerted by its external adhesive film that adheres to the skin in the periphery of the dressing (6); as well as the central negative tangential pressures (10) developed under the adherent surface of the dressing.

Figure 2 also indicates with arrows the negative pressures exerted by the orifices (9a) of the end of the redon catheters (9) inserted in the underlying surgical plane (8).

Having sufficiently described the nature of the present invention, as well as the manner of putting it into practice, it is not considered necessary to give greater explanation so that any person skilled in the art can understand its scope and the advantages derived therefrom, placing on record that, within is essential nature, it can be put into practice in other forms of embodiment that differ in details from those indicated by way of example, and whereto protection claimed will equally be given, provided that its main principle is not altered, changed or modified.

## Claims

1. Improved vacuum dressing applicable as post-operative compression treatment, sealed by an adhesive film (5) whereon is coupled a suction tube (4) connected to a vacuum pump, being of the type that comprises, under its sealing film, an external layer (2) of foam with open pores of a big size or macro pores (2a) and an internal layer (3), **characterized in that** it is an integrated dressing suitable for post-operative compression that makes is possible to perform negative tangential pressures not only in the periphery of the dressing (6), but also under the adherent surface thereof (10), with dimensions greater than conventional ones and with a certain thickness to favour compression and enable the incorporation of a drainage device, **in that** the internal layer (3) is semipermeable and adherent.

2. Improved vacuum dressing applicable as post-operative compression treatment, according to claim 1, **characterized in that** the improved vacuum dressing comprises a drainage device; wherein said device consists of redon catheters (9) whereto a plurality of holes (9a) has to be made at its two ends, of which ends, one is inserted in the external layer (2) of foam with macro pores of the dressing (1), and the opposite is designed to be inserted in the operative plane (8)

## Patentansprüche

1. Als postoperative Kompressionsbehandlung anwendbarer, verbesserter Vakuumverband, abgedichtet durch einen Klebefilm (5), dem ein Saugröhrchen (4) angeschlossen ist, das mit einer Vakuumpumpe verbunden und von der Art ist, der unter seiner Siegelfolie eine äußere Schicht (2) aus Schaum mit offenen großen Poren oder Makroporen (2a) und eine innere Schicht (3) umfasst, **dadurch gekennzeichnet, dass** es sich um eine integrierte Wundauflage handelt, die für eine postoperative Kompression geeignet ist, negative Tangentialdrücke nicht nur im Umfang des Verbandes (6), sondern auch unter dessen Haftfläche (10) zu ermöglichen, Abmessungen aufweist, die größer als herkömmliche sind und eine bestimmte Dicke hat, um die Kompression zu fördern und den Einbau einer Entwässerungsvorrichtung zu ermöglichen, indem die innere Schicht (3) semipermeabel und anhaftend ist.

2. Als postoperative Kompressionsbehandlung anwendbarer, verbesserter Vakuumverbandnach Anspruch 1, **dadurch gekennzeichnet, dass** der verbesserte Vakuumverband eine Entwässerungsvorrichtung umfasst; wobei die Vorrichtung aus Redonkathetern (9) besteht, an deren zwei Enden eine Vielzahl von Bohrungen (9a) angebracht werden, wobei eine von diesen Enden in die äußere Schicht (2) aus Schaumstoff (20) mit Makroporen des Verbandes (1) angebracht wird, und wobei das Gegenteil ausgestaltet ist, um in die operative Ebene (8) eingefügt zu werden.

## Revendications

1. Pansement à pression négative perfectionné utilisable comme traitement de compression postopératoire, scellé par un film adhésif (5) sur lequel est couplé un tube d'aspiration (4) raccordé à une pompe à vide, étant du type qui comprend, sous son film de scellement, une couche externe (2) de mousse avec pores ouverts de grande taille ou macropores (2a) et une couche interne (3), **caractérisé en ce qu'**il est un pansement intégré adapté à la compression postopératoire qui lui permet d'exercer des pressions négatives tangentielles non seulement à la périphérie du pansement (6), mais aussi sous sa surface d'adhésion (10), aux dimensions supérieures aux dimensions conventionnelles et d'une certaine épaisseur pour favoriser la compression et permettre l'intégration d'un dispositif de drainage, **en ce que** la couche interne (3) est semi-perméable et adhésive.

2. Pansement à pression négative perfectionné utilisable comme traitement de compression postopératoire, selon la revendication 1, **caractérisé en ce que** le pansement à pression négative perfectionné comprend un dispositif de drainage ; dans lequel ledit dispositif consiste en des cathéters de Redon (9) sur lesquels une pluralité de trous (9a) doit être réalisée à ses deux extrémités, extrémités dont l'une est insérée dans la couche externe (2) de mousse avec macropores du pansement (1), et l'extrémité opposée est conçue pour être insérée dans le plan opératoire (8).
